(19) Europäisches Patentamt
European Patent Office
Office européen des brevets

(11) Publication number : **0 591 051 A2**

# (12) EUROPEAN PATENT APPLICATION

(21) Application number : **93402379.7**

(22) Date of filing : **29.09.93**

(51) Int. Cl.$^5$ : **C12N 1/04**, A23B 7/10, A23B 7/154, A23B 7/155

(30) Priority : **30.09.92 JP 261470/92**
**26.04.93 JP 99407/93**

(43) Date of publication of application :
**06.04.94 Bulletin 94/14**

(84) Designated Contracting States :
**BE DE DK ES FR GB IE LU NL SE**

(71) Applicant : **Tsukuni, Hajime**
**17-7, Asagaya-Kita 2-chome, Suginami-ku**
**Tokyo (JP)**

(72) Inventor : **Ooshiro, Zentaro, c/o KOASA Central Lab.**
**1257, Aza-Kaminada, Kounominato, Genkai-Cho**
**Munakata-Gun, Fukuoka-Ken (JP)**

(74) Representative : **Desaix, Anne et al**
**Ernest Gutmann - Yves Plasseraud S.A. 3, rue Chauveau-Lagarde**
**F-75008 Paris (FR)**

## (54) Process for preserving raw algae and preserved food of raw algae.

(57) There are disclosed a process for preserving a raw alga belonging to red algae or brown algae, which comprises bringing the alga into contact with an aqueous solution containing at least one selected from the group consisting of an acid, lysozyme and ε-polylysine, and a preserved food of a raw alga which comprises a raw alga having been contacted with an aqueous solution containing at least one selected from the group consisting of an acid, lysozyme and ε-polylysine.

EP 0 591 051 A2

## BACKGROUND OF THE INVENTION

This invention relates to a process for preserving a raw alga and a preserved food of raw alga. More particularly, this invention relates to a process for preserving a raw alga belonging to red algae or brown algae, and to a preserved food using the same.

Generally, a raw alga having poor storability is known to rapidly lose its freshness, resulting in degradation of quality. For preventing this, the raw alga is freeze-preserved or subjected to a treatment such as salting or drying before it is come into the market.

Particularly in red algae such as *Porphyra tenera* (Asakusa laver), Ceylon moss and *Meristotheca papulosa,* and brown algae such as *Laminaria* spp. (kelp), *Undaria pinnatifida* (Undaria) and *Hizikia fusiformis* (Hizikia), quality thereof is rapidly degraded when they contain water. Therefore, an easy and useful process for preserving the alga has been long demanded.

Namely, in these raw algae, proteins or saccharides contained therein are decomposed, and simultaneously useful substances such as pigments or minerals are dissolved out therefrom because of self-digestion and bacterial decomposition when they contain water. Furthermore, they smell rotten. Therefore, the raw alga rapidly loses its value as a raw alga food with the time course.

Among various red algae, laver becomes self-digested and becomes rotten more shortly as compared with the other algae, and proteins, minerals, and chromoproteins such as chlorophyll, phycoerythrin and phycocyan dissolve out therefrom, when the laver contains water. Also, when thalli of laver are freeze-preserved, the thalli exhibit red rust-like color. These result in the difficulty in maintaining the original quality of the raw alga. Therefore, it has been impossible to preserve laver under raw conditions for a long time without spoiling the flavor, the touch on tongue, and the color tone of the laver thalli that the raw laver originally has.

As the laver food, laver sheets obtained by forming and drying raw alga of laver, so-called dried laver such as toasted laver obtained by toasting the laver sheets and seasoned laver, and Tsukudani, i.e., laver boiled with sea-sonings in soy, etc., are conventionally known. Such laver food has a preservability improved by lowering the water activities of the laver thalli.

However, laver which can be used as raw materials for the preserved food is limited to fresh and raw laver which has been just harvested. Therefore, the preserved food has a problem that the preserved food can be manufactured only during the period when the raw laver is harvested, and particularly has a problem that when raw laver for laver sheets is preserved over 10 hours, the manufactured laver sheet products lose the glosses.

## SUMMARY OF THE INVENTION

An object of the present invention is to provide a process for preserving a raw alga by which a raw alga can be preserved at a cool place or at room temperature for a long time without losing the taste, the touch on tongue and the color tone that raw red algae (particularly, raw laver and *Meristotheca papulosa)* and raw brown algae (particularly, *Undaria pinnatifida* and sporophyll thereof) originally have; and to provide a preserved food of raw alga using the same.

The first invention of the present invention is a process for preserving a raw alga belonging to red algae or brown algae, which comprises bringing the alga into contact with an aqueous solution containing at least one selected from the group consisting of an acid, lysozyme and ε-polylysine.

The second invention of the present invention is a preserved food of a raw alga which comprises a raw alga having been contacted with an aqueous solution containing at least one selected from the group consisting of an acid, lysozyme and ε-polylysine.

## BRIEF DESCRIPTION OF THE DRAWINGS

Fig. 1 is a graph showing a relation of the preservation period and the viable cell count.

Fig. 2 is a graph showing a relation of absorbance measured by a spectrophotometer and wavelength.

Fig. 3 is a graph showing a relation of the preservation period and the viable cell count.

Fig. 4 is a graph showing a relation of the preservation period and the viable cell count.

Fig. 5 is a graph showing a relation of absorbance measured by a spectrophotometer and wavelength.

Fig. 6 is a graph showing a relation of the preservation period and the viable cell count.

Fig. 7 is a graph showing a relation of the preservation period and the viable cell count.

Fig. 8 is a graph showing a relation of the preservation period and the viable cell count.

Fig. 9 is a graph showing a relation of absorbance measured by a spectrophotometer and wavelength.

## DESCRIPTION OF THE PREFERRED EMBODIMENT

In the following, the present invention is explained in detail.

Red algae usable in the present invention are soft algae which do not have distinguishable root, stem or leaf, are in the thread-like, leaf-like or feather-like form, contain phycobilin in addition to chlorophyll, and exhibit red or purple color. As a concrete example, there can be said an alga belonging to *Porphyra spp.* (purple laver) of red algae, called as a laver, such as *Porphyra tenera, Porphyra yezoen- sis, Porphyra angusta, Porphyra suborbiculata, Porphyra crispata, Porphyra okamurai, Porphyra pseudolinearis* and *Porphyra umbilicalis,* and an alternative thereof; Ceylon moss; and *Meristotheca papulosa.*

Brown algae usable in the present invention are called as brown algae because they contain brown pigment, and as a concrete example, there can be said *Laminaria* spp. (kelp), *Hizikia fusiformis* (hizikia) and *Undaria pinnatifida* (undaria) including a sporophyll thereof. The raw alga according to the present invention may include raw alga just having been harvested, and raw alga which has been cut into proper sizes by a chopper or so after harvested.

In order to carry out the process for preserving of the present invention, any method can be used as far as the raw alga is efficiently come into contact with the above aqueous solution by this method. For example, the raw alga is washed in water and dehydrated, and the above aqueous solution is sprinkled thereon; the raw alga having been dehydrated is immersed in the above aqueous solution; or at least one selected from an acid, lysozyme and ε-polylysine is added to the raw alga which has been soaked in water so that it is uniformly dissolved in the aqueous phase.

In the process for preserving of the present invention and the preserved food, described below, the raw alga can be come into contact with the above aqueous solution in a voluntary combination ratio as far as the raw alga is uniformly contacted with the aqueous solution, and is preferably contacted with the aqueous solution in a ratio of 2 to 5 g as a weight when dried, against 100 g of the aqueous solution.

An acid usable in the present invention may include an organic acid such as acetic acid, citric acid, tartaric acid, and succinic acid; and a mineral acid such as hydrochloric acid. Among them, acetic acid is preferable.

Lysozyme usable in the present invention may be in the form of salt such as lysozyme chloride.

ε-Polylysine usable in the present invention is represented by the formula below although it is a known compound:

$$H\left[NH-CH_2-CH_2-CH_2-CH_2-\underset{\displaystyle NH_2}{\underset{|}{CH}}-CO\right]_n OH$$

wherein n is preferably an integer of 20 to 30.

The acid is added so that the above aqueous solution may have a pH value of 6.5 to 2.0, preferably 5.5 to 3.0, more preferably 4.5 to 3.5. For example, when acetic acid is used, the concentration of acetic acid is 0.001 to 5.0 % by weight, preferably 0.01 to 2.5 % by weight, more preferably 0.1 to 1.0 % by weight.

Lysozyme is preferably added to the aqueous solution in an amount of 0.0001 to 0.01 % by weight, more preferably, 0.001 to 0.01 % by weight.

ε-Polylysine is preferably added to the aqueous solution in an amount of 0.0001 to 5.0 % by weight, more preferably 0.01 to 1.0 % by weight, the most preferably 0.05 to 0.5 % by weight.

In the process for preservation of the present invention, an acid, lysozyme or ε-polylysine can be used separately as above described, while the preservative effects can be synergistically increased by using them in combination, as described below. Particularly, the acid and lysozyme are preferably used together with each other.

For example, when an acid is used in combination with lysozyme, the combination ratio of lysozyme with the acid is 0.0001 to 0.01 % by weight, preferably 0.001 to 0.01 % by weight of lysozyme to such an amount of acid that the mixture has a pH value of 5.5 to 3.0. For example, acetic acid is used in an amount of 0.01 to 2.5 % by weight, preferably 0.1 to 1.0 % by weight. The ratio by weight of the acid to lysozyme is preferably 10 to 1000.

As another example, when an acid is used in combination with ε-polylysine, the combination ratio of ε-polylysine with the acid is 0.0001 to 5.0 % by weight, preferably 0.001 to 1.0 % by weight of ε-polylysine to such an amount of acid that the mixture has a pH value of 6.0 to 9.0, and for example, acetic acid is used in an amount of 0.00001 to 0.5 % by weight, preferably 0.001 to 0.1 % by weight. The ratio by weight of acid to ε-polylysine is preferably 0.01 to 1.

In the process for preserving of the present invention, when required, minerals such as sodium chloride and potassium chloride, an alcohol, a coloring agent, an antiseptic, and a seasoning can be added as well as the acid, lysozyme and ε-polylysine. Among them, sodium chloride is preferably added since the activation of lysozyme is accelerated by containing 0.4 to 0.6 % by weight of sodium chloride.

The raw alga maintained within the range of the above pH is preferably stored at 0 to 30 °C.

The preserved food of raw alga of the present invention is in the form of food obtained by the above process for preserving, and comprises a mixture of raw alga and an aqueous solution containing an acid, lysozyme or ε-polylysine separately or in combination with each other. Also, the preserved food of raw alga of the present invention can be a packed mixture.

As for the package form, the preserved food of raw laver can be packed in known package forms, and can be preferably packed in the form of sealed pack such as a bottle, a can, or a vacuumized pack.

By using the process for preserving of the present invention, a raw alga can be maintained fresh for a long time (at least 3 months, or 6 months depending on the preservation condition such as a temperature, a package form and an additive), and is available as a preserved food for sale. Such a stable raw alga as a product is quite novel, and the preserved food of the present invention enables an urban resident to obtain a fresh raw alga. The preserved food can be provided on the table as it is, and is also available as an ingredient of a salad, a soup or a vinegared dish.

It is not necessary to rapidly process a raw alga to prepare a processed food such as a dried alga (for example, dried laver), a seasoned raw alga (for example, seasoned laver) and Tsukudani, which contributes to the stable quality of the processed food and high processability.

## EXAMPLES

In the following, Examples of the present invention and Comparative Examples are described in detail. These Examples are not intended to limit the scope of the present invention at all.

### Example 1

A raw alga of *Porphyra yezoensis* was washed with clean sea water, followed by dehydration, and to 2 g of the moist raw alga having a moisture content of 80 %, 20 ml of 0.1 % acetic acid solution was added to prepare a preserved food of raw laver having a pH value of 3.5, and a polypropylene wide-mouthed bottle was filled with the preserved food and sealed.

On the other hand, a preserved food of raw laver was also prepared in the same manner as in the above except that distilled water was used in place of the acetic acid solution, and a polypropylene widemouthed bottle was filled with it and sealed, as a control.

The thus obtained preserved food of raw laver was preserved in a dark place at 15 °C for a prescribed period to evaluate the preservability by measuring the viable general cell count, detecting the chromoproteins, and carrying out a panel test.

### Measurement of Viable General Cell Count

The filtrate of the above preserved food of raw laver after preserved for a prescribed period, was properly diluted, and cultured in a standard agar medium at 37 °C for 48 hours to count the number of colonies. The results are shown in Fig. 1.

As clear from Fig. 1, the preserved food of raw laver of the present invention exhibited no increase in the viable cell count whereas the control exhibited an increase in the viable cell count.

### Detection of Chromoproteins

The filtrate of the above preserved food of raw laver, preserved for 14 days after the preparation, was scanned by a spectrophotometer (manufactured by Shimazu Co., UV 210) within the range of 400 to 700 nm of wavelength to detect the absorbance peaks. The results are shown in Fig. 2.

As clear from Fig. 2, the preserved food of raw laver of the present invention did not exhibit any absorbance peak due to dissolution of the chromoproteins, whereas the control exhibited the absorbance peaks.

### Panel Test

As for the preserved food of raw laver, preserved for 7 days after the preparation, the taste, the feeling

when eating, the color tone, and the savor were evaluated by 6 panelists with the two point-discrimination method (a preferred one obtains 1 point). The results are shown in Table 1.

Table 1

| | Example 1 | Control |
|---|---|---|
| Taste | 6 | - |
| Feeling when eating | 6 | - |
| Color tone | 6 | 0 |
| Savor | 6 | 0 |

-: rotted

As clear from Table 1, the preserved food of raw laver of the present invention gained higher evaluation.

## Example 2

A preserved food of raw laver having a pH value of 3.7 was prepared in the same manner as in Example 1 except that 20 ml of a mixture solution of 0.1 % acetic acid and 0.01 % lysozyme was used in place of 20 ml of 0.1 % acetic acid solution, and a polypropylene widemouthed bottle was filled with the preserved food and sealed. The control used was the same as in Example 1.

The preservability of the thus obtained preserved food of raw laver was evaluated in the same manner as in Example 1. The results of measurement of the viable general cell count, and the panel test are shown in Fig. 3 and Table 2, respectively.

As clear from Fig. 3, the preserved food of raw laver of the present invention exhibited no increase in the viable cell count whereas the control exhibited an increase in the viable cell count.

Table 2

| | Example 2 | Control |
|---|---|---|
| Color tone | 6 | 0 |
| Savor | 6 | 0 |

As clear from Table 2, the preserved food of raw laver of the present invention gained higher evaluation.

## Example 3

A raw alga of *Porphyra yezoensis* was washed with clean sea water, followed by dehydration, and to 3 g of the moist raw alga having a moisture content of 80 %, 30 ml of a 0.5 % by weight aqueous solution of ε-polylysine having a pH value of 9.5 was added to prepare a preserved food of raw laver, and a polypropylene widemouthed bottle was filled with the preserved food and sealed.

The thus obtained preserved food of raw laver was preserved in a dark place at 15 °C for a prescribed period to evaluate the preservability by measuring the viable general cell count, detecting the chromoproteins, and carrying out a panel test.

### Measurement of Viable General Cell Count

The number of colonies was counted in the same manner as in Example 1. The results are shown in Fig. 4.

Detection of Chromoproteins

The absorbance peaks of chromoproteins were detected in the same manner as in Example 1 except that the filtrate of the above preserved food of raw laver, preserved for 38 days after the preparation was used. The results are shown in Fig. 5.

Panel Test

As for the preserved food of raw laver, preserved for 19 days after the preparation, the taste, the feeling when eating, the color tone, and the savor were evaluated by 6 panelists according to the following criteria:
The highest quality: 5 points
The lowest quality: 0 points
The results are shown in Table 3.

Example 4

A preserved food of raw laver was prepared to evaluate the preservability by measuring the viable general cell count, detecting the chromoproteins, and carrying out a functional test, in the same manner as in Example 3 except that 30 ml of an aqueous solution having a pH value of 8.6, containing 0.01 % by weight of acetic acid and 0.5 % by weight of ε-polylysine was used in place of an aqueous solution containing 0.5 % by weight of ε-polylysine. The results are shown in Fig. 4, Fig. 5 or Table 3.

Comparative Example 1

A preserved food of raw laver was prepared to evaluate the preservability by measuring the viable general cell count, detecting the chromoproteins, and carrying out a panel test, in the same manner as in Example 1 except that 30 ml of distilled water was used in place of an aqueous solution containing 0.5 % by weight of ε-polylysine. The results are shown in Fig. 4, Fig. 5 or Table 3.

Table 3

| | Example 3 | Example 4 | Comparative Example 1 |
|---|---|---|---|
| Taste | 1.6 | 4.0 | - |
| Feeling when eating | 1.8 | 3.8 | - |
| Color tone | 1.5 | 4.8 | 0 |
| Savor | 2.3 | 4.3 | 0 |

-: rotten

Evaluation

As clear from Fig. 4, the preserved food of raw laver of Example 3 exhibited an extremely slight increase in the viable cell count as compared with that of Comparative Example 1. The preserved food of raw laver of Example 4 exhibited no increase in the viable cell count.

As clear from Fig. 5, the preserved food of raw laver of Examples 3 and 4 exhibited little absorption peak due to dissolution of the chromoproteins whereas the preserved food of raw laver of Comparative Example 1 exhibited absorption peaks.

As clear from Table 3, the preserved food of raw laver of Examples 3 and 4 gained higher evaluation as compared with that of the preserved food of raw laver of Comparative Example 1.

Example 5

Four grams of *Meristotheca papulosa,* 6 g of *Undaria pinnatifida* and 7 g of sporophyll of *Undaria pinnatifida,* each in the raw form, were washed twice with an acetic acid solution having a concentration of 0.01 %, 0.025 %, 0.05 %, 0.1 %, 0.25 %, 0.5 % or 1.0 %, prepared as a preservation solution of the alga, and a glass bottle was filled with the alga together with 100 ml of the preservation solution, and sealed as a preserved food of raw alga. For the control, distilled water was used. Sea water was also used.

The thus obtained preserved food of raw alga was preserved in a dark place at 15 °C for a prescribed period. Then, the preservability of the raw alga was evaluated by measuring the pH and the viable general cell count, as described below. As for *Meristotheca papulosa,* the preservability was also evaluated by detecting the chromoproteins in the preservation solution.

pH

The pH values of the preserved food of the above described *Meristotheca papulosa, Undaria pinnatifida,* and sporophyll of *Undaria pinnatifida,* preserved for a prescribed period after the preparation were measured. The results are shown in Tables 4 through 6.

Table 4

*Meristotheca papulosa*

| | | | Preservation period (day) | | | |
|---|---|---|---|---|---|---|
| | | | 0 | 9 | 16 | 55 |
| Acetic acid conc. (%) | 0 | ① | 6.59 | 6.15 | 6.19 | 5.80 |
| | 0.01 | ② | 4.66 | 4.64 | 4.77 | 4.77 |
| | 0.025 | ③ | 4.17 | 4.19 | 4.17 | 4.35 |
| | 0.05 | ④ | 3.90 | 3.93 | 3.96 | 3.98 |
| | 0.01 | ⑤ | 3.70 | 3.80 | 3.84 | 3.69 |
| | 0.25 | ⑥ | 3.37 | 3.40 | 3.44 | 3.37 |
| | 0.5 | ⑦ | 3.20 | 3.33 | 3.25 | 3.12 |
| | 1.0 | ⑧ | 2.88 | 2.94 | 2.97 | 2.96 |
| Sea water | | ⑨ | 6.75 | 6.23 | 6.29 | 6.37 |

Table 5

*Undaria pinnatifida*

| | | | Preservation period (day) | | | |
|---|---|---|---|---|---|---|
| | | | 0 | 9 | 16 | 55 |
| Acetic acid conc. (%) | 0 | ① | 5.89 | 5.27 | 5.79 | 5.19 |
| | 0.01 | ② | 4.47 | 4.51 | 4.58 | 4.75 |
| | 0.025 | ③ | 4.20 | 4.26 | 4.28 | 4.22 |
| | 0.05 | ④ | 3.94 | 4.01 | 4.01 | 4.00 |
| | 0.01 | ⑤ | 3.73 | 3.76 | 3.80 | 3.73 |
| | 0.25 | ⑥ | 3.53 | 3.51 | 3.55 | 3.53 |
| | 0.5 | ⑦ | 3.33 | 3.38 | 3.33 | 3.28 |
| | 1.0 | ⑧ | 3.07 | 3.90 | 3.09 | 3.07 |
| Sea water | | ⑨ | 6.52 | 4.46 | 4.28 | 4.31 |

Table 6

Sporophyll of *Undaria pinnatifida*

| | | | Preservation period (day) | | | |
|---|---|---|---|---|---|---|
| | | | 0 | 9 | 16 | 55 |
| Acetic acid conc. (%) | 0 | ① | 5.51 | 5.27 | 5.25 | 5.12 |
| | 0.01 | ② | 4.78 | 4.94 | 5.02 | 5.08 |
| | 0.025 | ③ | 4.37 | 4.54 | 4.49 | 4.48 |
| | 0.05 | ④ | 4.24 | 4.30 | 4.29 | 4.20 |
| | 0.01 | ⑤ | 3.98 | 4.50 | 4.04 | 3.97 |
| | 0.25 | ⑥ | 3.80 | 3.73 | 3.76 | 3.68 |
| | 0.5 | ⑦ | 3.48 | 3.54 | 3.54 | 3.43 |
| | 1.0 | ⑧ | 3.30 | 3.33 | 3.32 | 3.25 |
| Sea water | | ⑨ | 6.26 | 4.24 | 4.29 | 4.68 |

As clear from Tables 4 through 6, the preserved food of raw alga of the present invention exhibited only a slight change in the pH value whereas that preserved in distilled water, as the control, or the sea water exhibited a decrease of the pH value. In the range of 0.025 % or more of acetic acid concentration, the pH value was almost constant.

Viable General Cell Count

The number of colonies were measured in the same manner as in Example 1. The results are shown in

Figs. 6 through 8. In the figures, ① through ⑧ represent the viable cell count when preserved in distilled water, 0.025 %, 0.05 %, 0.1 %, 0.25 %, 0.5 % and 1.0 % acetic acid solutions, and sea water, respectively.

As clear from Figs. 6 through 8, the preserved food of raw alga of the present invention exhibited little increase in the viable cell count in the range of 0.1 % or more of acetic acid concentration whereas, in the control or in case where sea water was used, an increase in the viable cell count was observed.

Detection of Chromoproteins

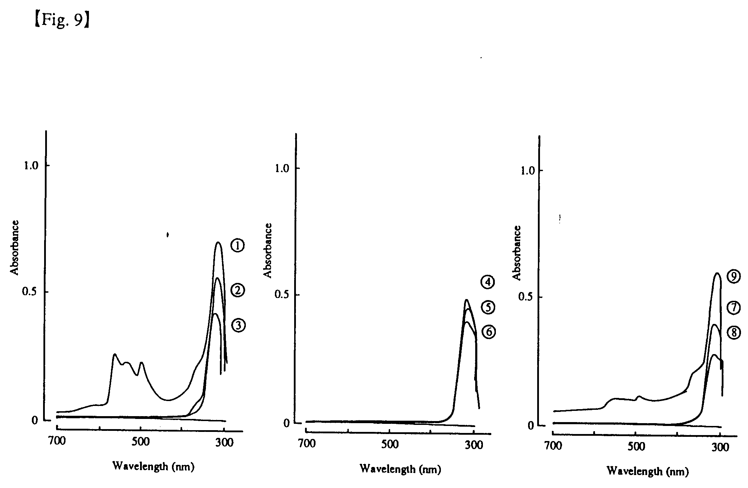

The absorbance peaks due to dissolution of the chromoproteins were detected in the same manner as in Example 1 except that the filtrate of the above preserved food of raw alga using *Meristotheca papulosa,* preserved for 16 days after the preparation was scanned by a spectrophotometer within the range of 300 to 700 nm of wavelength. The results are shown in Fig. 9. In the figure, ① through ⑨ represent the absorbance when preserved in distilled water, 0.01 %, 0.025 %, 0.05 %, 0.1 %, 0.25 %, 0.5 % and 1.0 % acetic acid solutions, and sea water, respectively.

As clear from Fig. 9, the preserved food of raw alga of the present invention exhibited no absorbance due to dissolution of the chromoproteins. On the other hand, the control employing distilled water exhibited a clear peak due to dissolution of the chromoproteins, and in case where sea water was used, slight absorption was observed.

Example 6

Raw alga of laver in an amount of 5.6 kg was immersed in 35 kg of 0.05 % ε-polylysine solution in 0.01 % acetic acid having a pH value of 8.2 for 3 hours, and by forming and drying the raw alga, laver sheets were prepared according to a conventional method.

The thus obtained laver sheets were evaluated by a panel test together with those prepared just after harvested (Control 1) and those prepared after immersed in sea water for the same period as the above (Control 2).

Each item was evaluated on the basis of 5 scores by 6 panelists, and the result was indicated as a mean value. The results are shown in Table 7.

Table 7

| | Example 6 | Control 1 | Control 2 |
|---|---|---|---|
| Color | 3.5 | 3.7 | 3.0 |
| Gloss | 4.0 | 3.8 | 2.6 |
| Flavor | 3.0 | 2.8 | 2.8 |
| Taste | 3.2 | 3.2 | 3.2 |

As clear from Table 7, the laver sheets of Example 6 exhibited glosses comparable to those of Control 1, and more excellent glosses than those of Control 2.

The process for preserving raw alga and the preserved food of raw alga according to the present invention have the following effects:

1. A raw alga, particularly raw laver, can be preserved for a long time without losing the taste, the touch on tongue, and the color tone that the raw alga originally has, and therefore, the raw alga can be stably supplied regardless of season.
2. As an acid is added only in a small amount, or as no acid is added, the taste of the raw alga is not affected. When the raw alga is used as raw materials for a dried alga, an apparatus for drying is hardly affected by corrosion in a process for drying the raw alga.
3. A novel preserved food of raw alga can be provided.
4. As a processed food of alga can be manufactured regardless of the harvesting period of the alga, the processed food of alga can be stably supplied.

## Claims

1. A process for preserving a raw alga belonging to red algae or brown algae, which comprises bringing the alga into contact with an aqueous solution containing at least one selected from the group consisting of an acid, lysozyme and ε-polylysine.

2. The process according to Claim 1 wherein the red algae belong to red algae of *Porphyra* spp.

3. The process according to Claim 1 wherein the pH of the aqueous solution is in the range of 6.5 to 2.0.

4. The process according to Claim 3 wherein the pH of the aqueous solution is in the range of 5.5 to 3.0.

5. The process according to Claim 4 wherein the pH of the aqueous solution is in the range of 4.5 to 3.5.

6. The process according to Claim 1 wherein the acid is acetic acid.

7. The process according to Claim 1 wherein the aqueous solution is an aqueous solution of acetic acid and lysozyme.

8. The process according to Claim 7 wherein the aqueous solution contains 0.0001 to 0.01 % by weight of acetic acid and the content by weight of the acetic acid is 10 to 1000 times that of the lysozyme.

9. The process according to Claim 1 wherein the aqueous solution is an aqueous solution of acetic acid and ε-polylysine.

10. The process according to Claim 9 wherein the aqueous solution contains 0.00001 to 0.5 % by weight of acetic acid and the content by weight of the acetic acid is 0.01 to 1 time that of the ε-polylysine.

11. A preserved food of a raw alga preserved by the process according to any one of Claims 1 through 10.

12. Stable, preserved food of a raw alga characterized in that it is constituted by said raw alga in admixture with an aqueous solution containing at least one component selected from the group consisting of an acid, lysozyme and ε-polylysine or a mixture thereof.

13. Stable preserved food of a raw alga, the aqueous phase of which is substantially free of chromoprotein, the release of which is substantially preventable by the contacting with said alga in a non-stabilized form with an aqueous solution as defined in any of claims 1 to 10.

14. Stable preserved food according to claim 12 or 13, wherein said raw alga belongs either to red algae, such as red algae of Porphyra spp., or brown algae.

【Fig. 1】

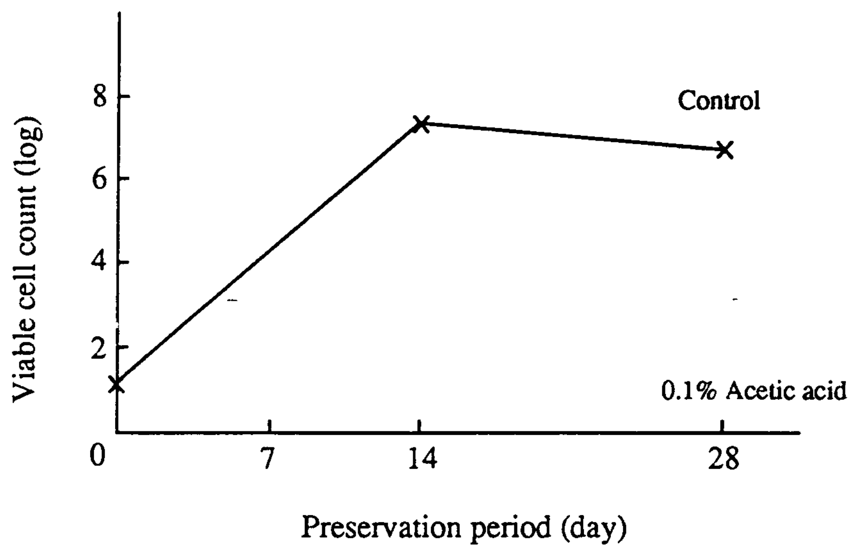
Viable cell count (log)
8
6
4
2
0
7
14
28
Control
0.1% Acetic acid
Preservation period (day)

【Fig. 2】

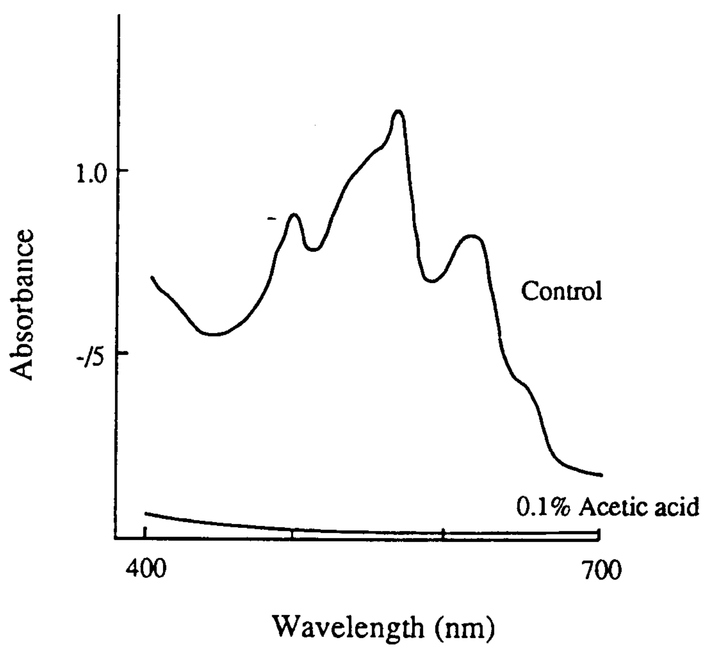
Absorbance
1.0
-/5
Control
0.1% Acetic acid
400
700
Wavelength (nm)

【Fig. 3】

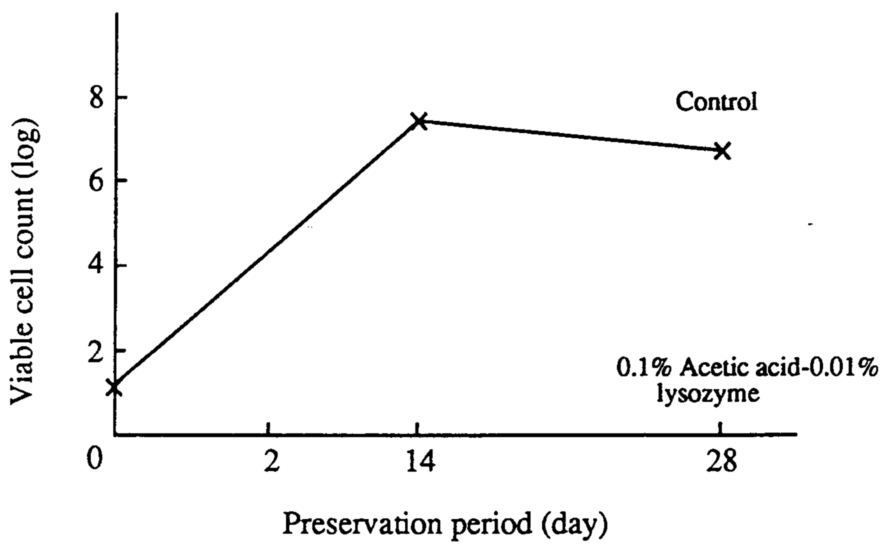
Viable cell count (log)
8
6
4
2
0
2
14
28
Preservation period (day)
Control
0.1% Acetic acid-0.01%
lysozyme

【Fig. 4】

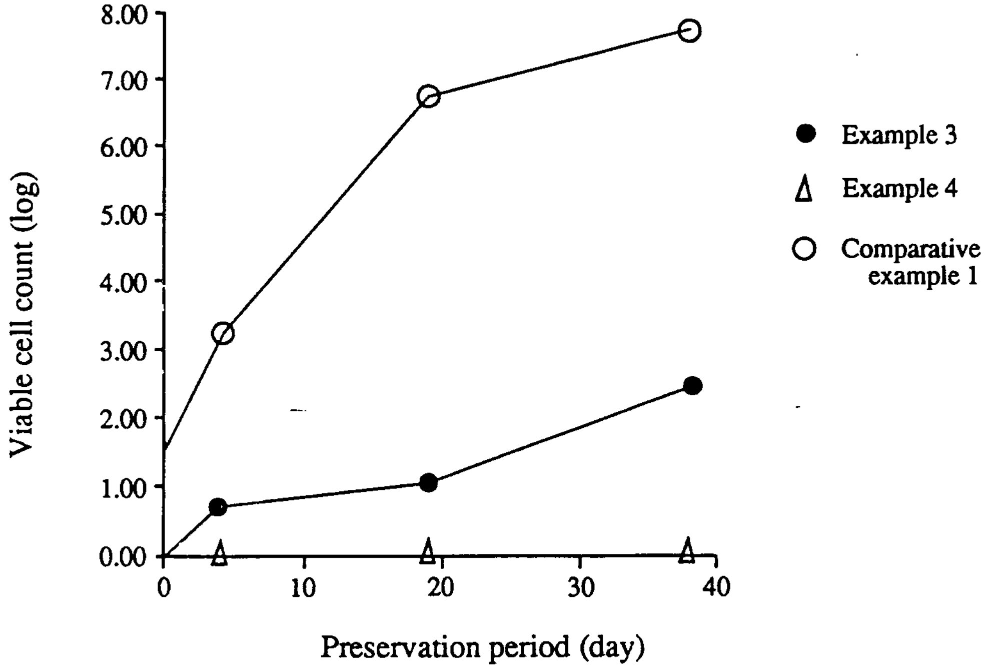
8.00
7.00
6.00
5.00
4.00
3.00
2.00
1.00
0.00
Viable cell count (log)
0
10
20
30
40
Preservation period (day)
Example 3
Example 4
Comparative example 1

【Fig. 5】

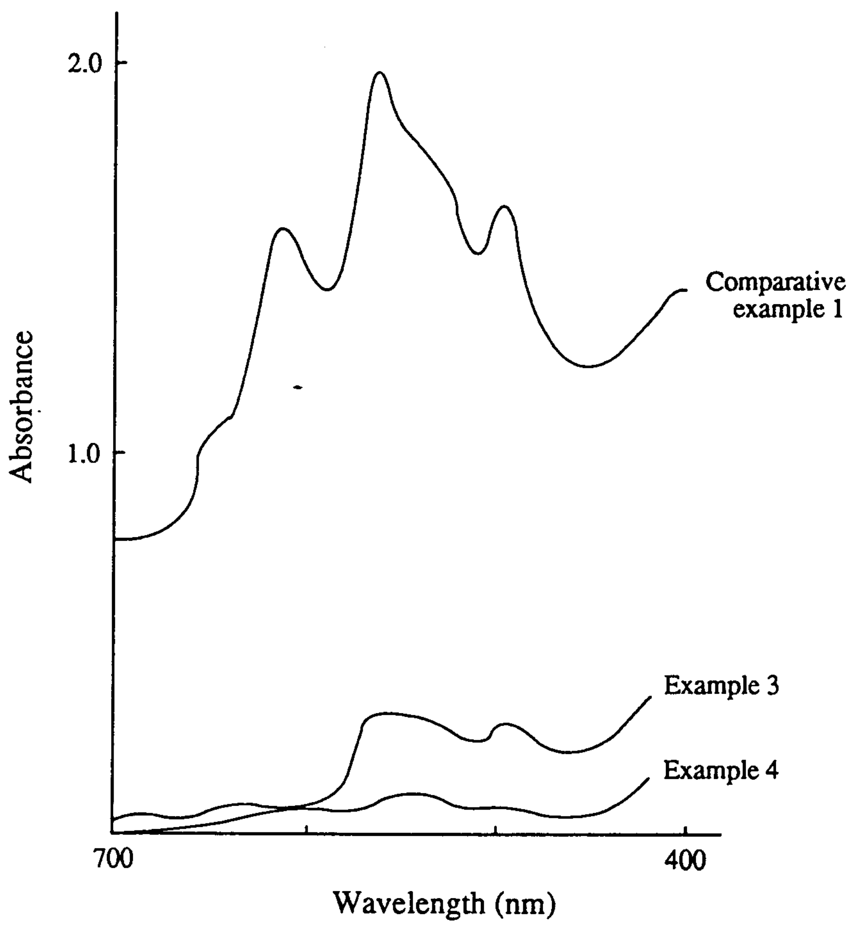
2.0
1.0
Absorbance
700
400
Wavelength (nm)
Comparative example 1
Example 3
Example 4

【Fig. 6】

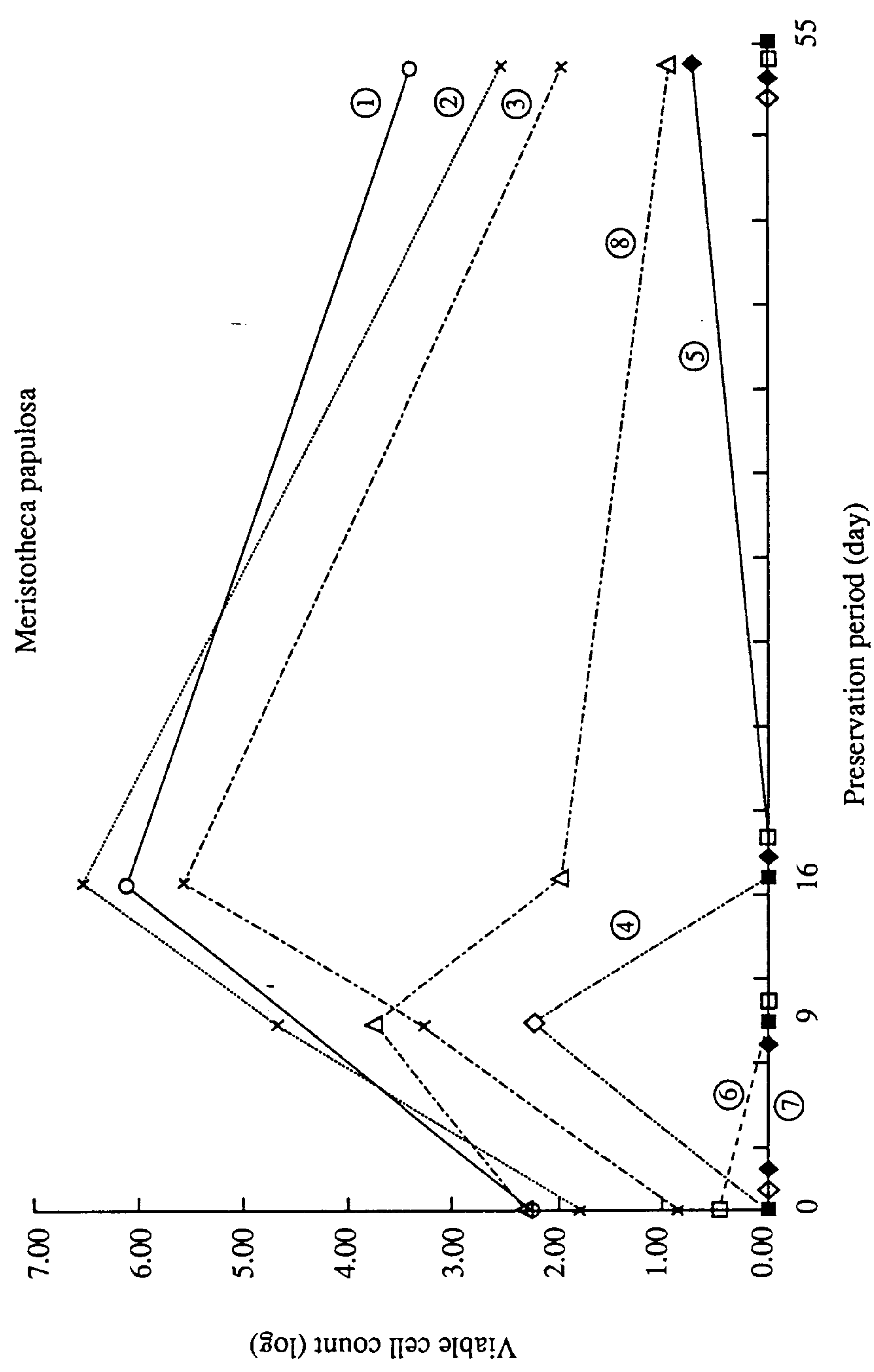
Meristotheca papulosa
Viable cell count (log)
7.00
6.00
5.00
4.00
3.00
2.00
1.00
0.00
0
9
16
55
Preservation period (day)
①
②
③
④
⑤
⑥
⑦
⑧

【Fig. 7】

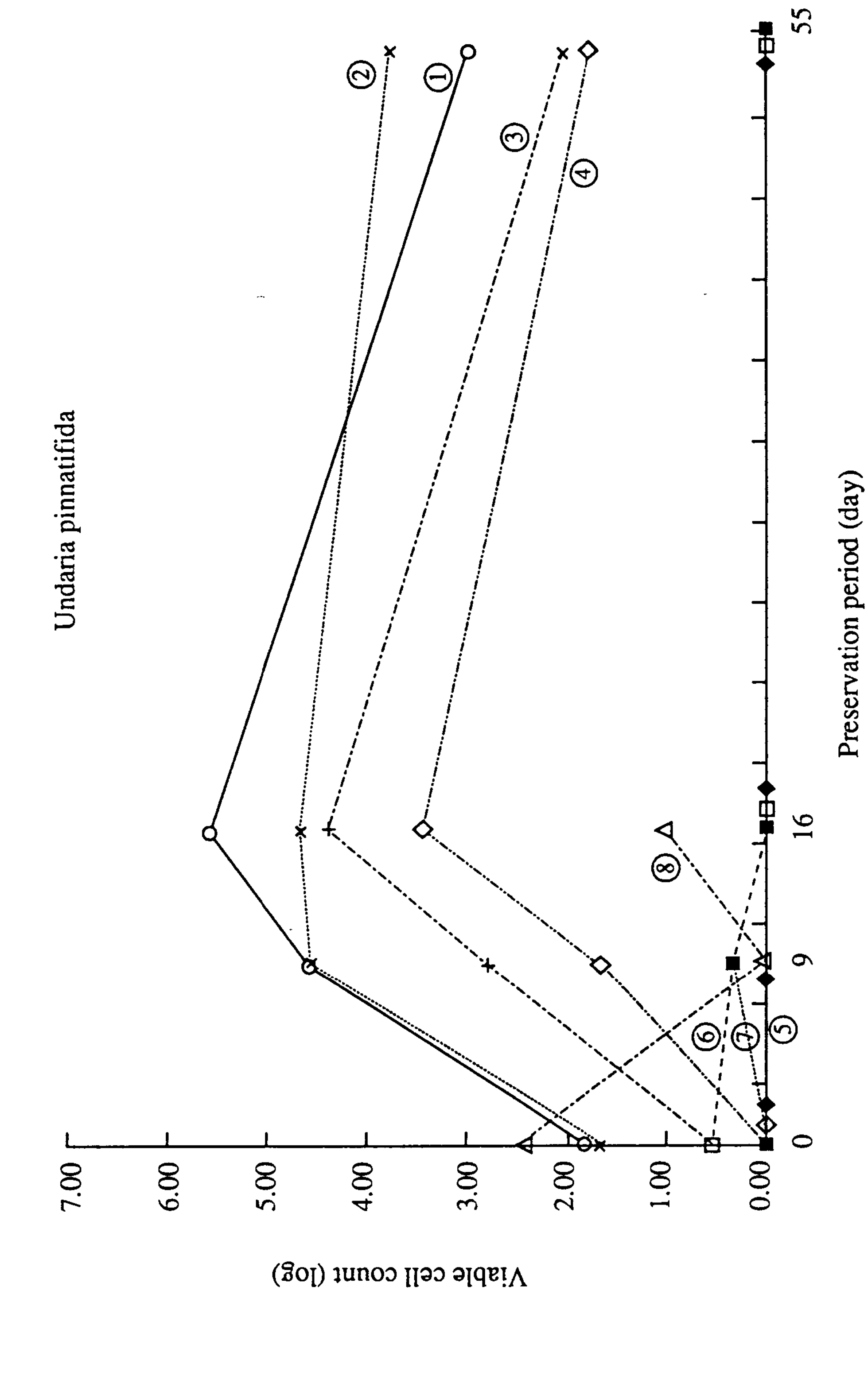
Undaria pinnatifida
Viable cell count (log)
7.00
6.00
5.00
4.00
3.00
2.00
1.00
0.00
0
9
16
55
Preservation period (day)
①
②
③
④
⑤
⑥
⑦
⑧

【Fig. 8】

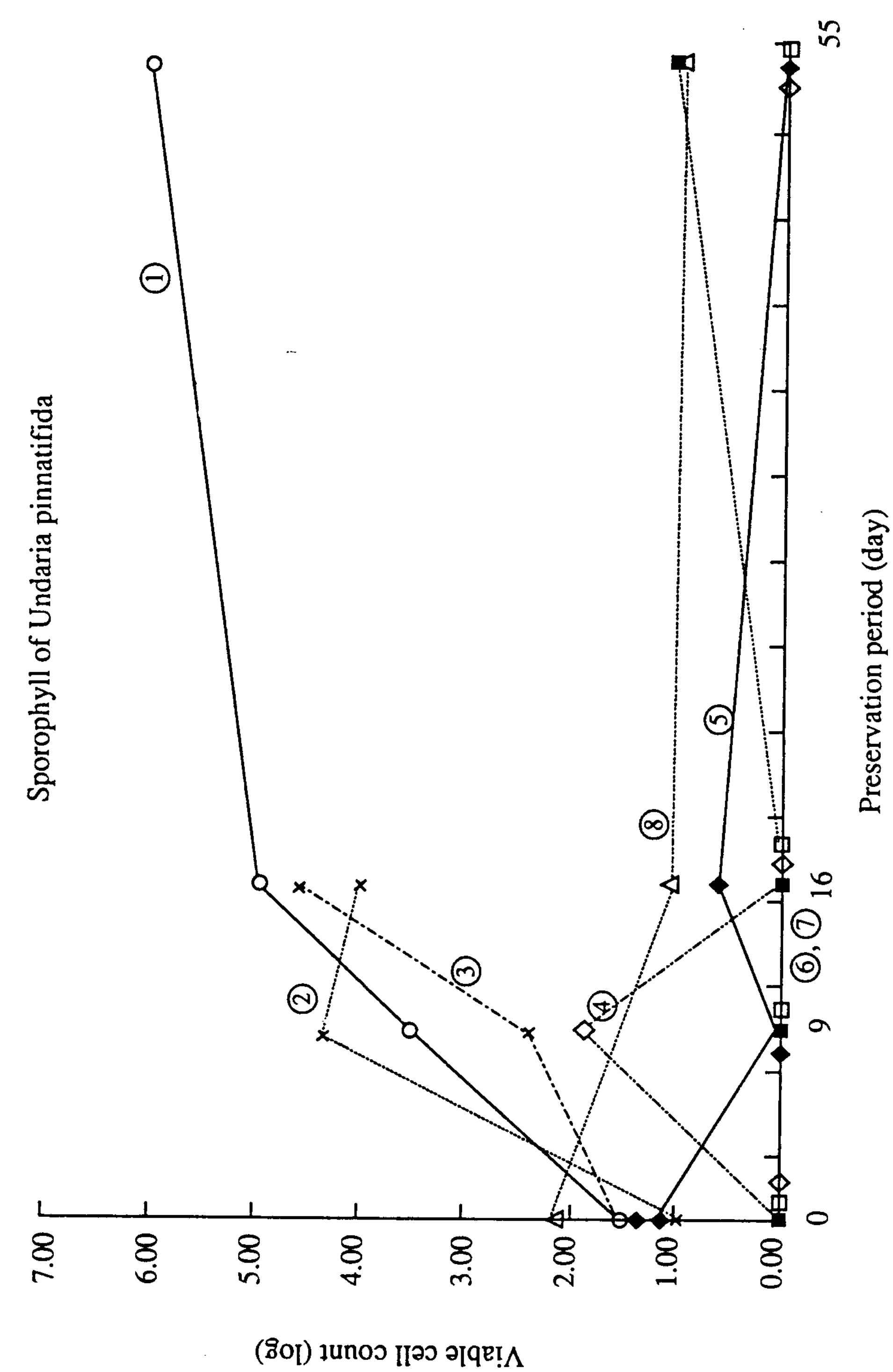
Sporophyll of Undaria pinnatifida
Viable cell count (log)
7.00
6.00
5.00
4.00
3.00
2.00
1.00
0.00
0
9
16
55
Preservation period (day)
①
②
③
④
⑤
⑥, ⑦
⑧

【Fig. 9】